# EUROPÄISCHE PATENTSCHRIFT

(11) **EP 0 956 004 B1**
(45) Veröffentlichungstag und Bekanntmachung des Hinweises auf die Patenterteilung: **15.09.2004**
(21) Anmeldenummer: 97948859.0
(22) Anmeldetag: 03.11.1997
(51) Int. Cl.: A61K 9/20

(54) **VERFAHREN ZUR HERSTELLUNG VON GEFORMTEN ODER UNGEFORMTEN POLYOLMASSEN UND HERGESTELLE ZUSAMMENSETZUNGEN**
METHOD FOR PRODUCING SHAPED AND UNSHAPED POLYOL MASSES AND COMPOSITIONS OBTAINED BY THE PROCESS
PROCEDE DE PREPARATION DE COMPOSITIONS POLYOL FACONNEES OU NON FAC ONNEES ET COMPOSITIONS OBTENUES

(30) Priorität: 15.11.1996 DE 19647282; 06.10.1997 DE 19743986
(43) Veröffentlichungstag der Anmeldung: 17.11.1999
(73) Patentinhaber: MERCK PATENT GmbH, 64293 Darmstadt (DE)
(72) Erfinder: MAUL, Karin, White Plains New York 10605 (US); SCHWARZ, Eugen, D-64625 Bensheim (DE)
(86) Internationale Anmeldenummer: PCT/EP1997/006046
(87) Internationale Veröffentlichungsnummer: WO 1998/022094

(56) Entgegenhaltungen:
- DE-A- 4 439 858
- DE-A- 19 509 805
- DE-A- 19 615 418
- DE-C- 3 506 276

## Beschreibung

Die Erfindung betrifft eine Zusammensetzung zur Herstellung von Tabletten, Komprimaten oder Lutschbonbons, enthaltend mindestens zwei Polyole, sowie gegebenenfalls ein oder mehrere Kohlenhydrate/e aus der Gruppe Stärke und Cellulose, gegebenenfalls einen oder mehrere Wirkstoffe, einen oder mehrere Farbstoffe, einen oder mehrere natürliche Süßstoffe, einen oder mehrere synthetische Süßstoffe, Säuerungsmittel, Geschmacksstoffe, Aromastoffe, sowie übliche Hilfstoffe,
herstellbar durch
a) Co-Versprühen von mindestens zwei Polyolen, gegebenenfalls mit löslichen Zusätzen, und
b) Extrusion in einem Temperaturbereich von 40 bis 110 °C in der gewünschten Zusammensetzung zu plastischen formbaren Massen, wobei
c) die Polyolzusammensetzungen entweder vor oder während des Extrudierens mit den unlöslichen Komponenten vermischt werden.

Die Erfindung betrifft auch ein Verfahren zur Herstellung einer Zusammensetzung, dadurch gekennzeichnet, daß mindestens zwei Polyole,welche zuvor, gegebenenfalls in Gegenwart von löslichen Zusätzen,cogesprüht worden sind, und gegebenenfalls ein oder mehrere Kohlenhydrate, gegebenenfalls ein oder mehrere Wirkstoffe, ein oder mehrere Farbstoffe, ein oder mehrere natürliche Süßstoffe, ein oder mehrere synthetische Süßstoffe, Säuerungsmittel, Geschmacksstoffe, Aromastoffe, Hilfstoffe in einem Temperaturbereich von 30 bis 170 °C, insbesondere in einem Bereich von 40 bis 110 °C extrudiert und gegebenenfalls die durch Extrudieren hergestellte plastische Masse, welche in Form eines Stranges vorliegen kann, in nachgeschalteten formgebenden Anlagen geformt werden und weiterverarbeitet wird.

Aus zahlreichen Veröffentlichungen und Patentanmeldungen sind Zusammensetzungen zur Herstellung Tabletten, Komprimaten oder auch Lutschbonbons bekannt (EP-A- 0 240 773, EP-A-0 462 066 oder DE-A1-43 16 537), in denen die verschiedensten physiologisch verträglichen Stoffe als Trägersubstanzen für pharmazeutische Wirkstoffe oder Geschmackstoffe dienen. Hierzu zählen insbesondere Cellulosederivate und deren Salze, Kohlehydrate, Zucker, wasserlösliche Polymere wie N-Vinylpyrrolidon-Vinylacetat-Copolymere, Polyvinylpyrrolidon, Polyvinylalkohol, Polyacrylsäure und ihre Salze, Polymethacrylsäure und ihre Salze, Polyalkylenoxide wie Polyethylenoxid, Polypropylenoxid sowie Copolymere aus Ethylen- und Propylenoxid, Polysaccharide wie Alginsäure, ihre Alkali- und Ammoniumsalze, Carrageenane, Galaktomannane, Traganth, Agar- Agar, Gummi arabicum, Xanthan gummi, Chitinderivate, wie Chitosan, Pektine, wie Natriumcarboxymethylamylpektin und Stärken sowie Gemische dieser wasserlöslichen Polymeren. Unter wasserlöslich ist hierbei zu verstehen, daß sich bei 20 °C in 100 g Wasser mindestens 0,5 g, bevorzugt 2 g des Polymeren gegebenenfalls koloidal auflösen bzw. unter Gelbildung lösen.

Eine gute Wasserlöslichkeit der Trägermatrix ist bei der Herstellung von Tabletten, Dragees, Lutschtabletten oder -bonbons von besonderer Bedeutung für die Bioverfügbarkeit und die schnelle Resorption.

Für ein angenehmes Geschmacks- und Mundgefühl ist zwar auch die Wasserlöslichkeit von Bedeutung, eine größere Rolle spielt in diesem Zusammenhang jedoch die Oberflächenbeschaffenheit und das Geschmacksempfinden während des Lutschens. Dieses ist bei den bekannten Trägermaterialien nicht ohne weiteres gegeben. Während die einen ein negatives Geschmacksbild besitzen, führen die anderen aufgrund ihrer physikalischen Beschaffenheit zu ungleichmäßigen, körnigen oder aufgrund ihrer kurzen Verarbeitbarkeit zu unebenen, gegebenenfalls scharfkantigen Oberflächen.

Üblicherweise werden, um eine homogene Verteilung eines zugefügten Wirkstoffs in der Trägermatrix zu erzielen, Vormischungen hergestellt, die verschiedenen Komponenten miteinander verschmolzen oder die Wirkstoffe durch Kneten in eine vorliegende Polymerschmelze gemischt. Probleme stellen bei diesen Verfahren die gleichmäßige Dosierung, die homogene Vermischung und die kontinuierliche Durchführung dar.

Durch DE-A-44 39 858 werden Zusammensetzungen vorbeschrieben, erhältlich durch Lösen von Polyolen in Wasser, Sprühtrocknen bei einer Temperatur von 120 bis 300 °C, Isolieren der Polyolzusammensetzungen, Vermischen mit weiteren Zusätzen und Tablettieren oder Weiterverarbeiten zu Kaugummis. Es werden also Endprodukte erhalten, die in sich eine inhomogene Struktur aufweisen. Darin enthaltene vermischte pulverförmige Zusätze werden zur Herstellung dieser Endprodukte während der Tablettierens durch das Verpressen lediglich miteinander verbunden, sind aber im Produkt bei der optischen Untersuchung noch partikelförmig erkennbar und beeinflussen das sensorische Mundgefühl.

In DE-A-35 06 276 werden Zusammensetzungen beschrieben, die hergestellt werden, indem Lactose in Wasser gelöst wird, zu der heißen Lösung Cellulosepulver hinzugegeben wird, abgekühlt wird und die erhaltene Masse granuliert und getrocknet wird, oder eine Aufschlämmung aus Lactose und Cellulose hergestellt wird, die anschließend sprühgetrocknet wird. Auch wenn durch das Letztere eine Sprühtrocknung mit eingebracht wird, werden durch diesen Arbeitsgang keine in sich homogenen Partikel erhalten, da die Einzelkomponenten nicht gelöst werden. Die Granulate weisen damit sowohl die Eigenschaften der gelösten als auch der ungelösten Komponenten auf.

Durch DE-1 95 09 805 werden Zubereitungen offenbart, die durch Extrusion einer Schmelze und anschließende Formgebung erhalten werden. Die Extrusion der Schmelze erfolgt nach dem Vermischen der Einzelkomponenten.

Um eine gleichbleibende Dosierung zu garantieren, muß zur Verabreichung von pharmazeutischen Wirkstoffen in Tabletten-, Drageeoder Lutschtablettenform der Wirkstoff in der Trägermatrix homogen verteilt sein. Dieses ist eine besonderes Problem bei der Verwendung von schwer löslichen Wirkstoffen.

Aufgabe der Erfindung ist es daher, einerseits eine Zusammensetzung zur Verfügung zu stellen, die schonend in einem Temperaturbereich zu den gewünschten Produkten, d. h. zu Tabletten, Komprimaten oder Lutschbonbons, verarbeitet werden können, in dem zugefügte Wirkstoffe nicht geschädigt werden. Aufgabe der Erfindung ist es auch, ein kontinuierlich durchführbares Verfahren zur Verfügung zu stellen, wodurch Tabletten, Komprimate, Lutschtabletten oder - bonbons hergestellt werden können, die eine glatte Oberfläche, welche auch während des Lutschens erhalten bleibt, und ein angenehmes Geschmacks- und Mundgefühl sowie eine homogene Verteilung darin enthaltener Wirk- und Aromastoffe aufweisen. Weiterhin ist es Aufgabe der Erfindung , Zusammensetzungen zur Verfügung zu stellen, welche sich in diesem Verfahren einsetzbar sind und durch eine lange Verformbarkeit in einfacher Weise zu den gewünschten Produkten verarbeiten lassen.

Die Lösung der Aufgabe erfolgt durch zuvor co-gesprühte Polyol enthaltende Zusammensetzungen, insbesondere durch Zusammensetzungen, enthaltend mindestens zwei Polyol/en aus der Gruppe Xylit, Sorbit oder Lactit, Maltit, Erythrit oder Mannit, und gegebenenfalls Kohlenhydrate aus der Gruppe Stärke, Cellulose , sowie je nach Produkt gegebenenfalls einen oder mehrere Wirkstoffe, einen oder mehrere Farbstoffe, einen oder mehrere natürliche Süßstoffe, einen oder mehrere synthetische Süßstoffe, Säuerungsmittel, Geschmacksstoffe, Aromastoffe sowie übliche Hilfstoffe.

Die Lösung der Aufgabe erfolgt auch durch ein Verfahren zur Herstellung einer plastischen, geformten oder ungeformten Masse in dem eine vorwiegend aus mindestens zwei Polyol Polyolen bestehende Zusammensetzung in einem Temperaturbereich von 30 bis 170°C extrudiert und gegebenenfalls geformt wird. Insbesondere erfolgt die Lösung der Aufgabe durch zuvor co-gesprühte Zusammensetzungen, die extrudiert werden und der auf diese Weise erhaltene Strang anschließend in nachgeschalteten formgebenden Anlagen weiterverarbeitet wird.

Es ist bekannt, zur Herstellung von lutschbaren Tabletten, Komprimaten oder Bonbons Mischungen der Einzelkomponenten zu extrudieren und unter erwärmen auf Temperaturen von unterhalb von 200 °C miteinander zu verschmelzen. Das Extrudat kann durch Kalandrieren oder durch Zerkleinern mit rotierenden Messern in volumengleiche, noch formbare Stücke mit erstarrter Oberfläche geteilt werden, welche direkt anschließend durch Verpressen zu Tabletten verarbeitet werden können. Es ist auch bekannt, über geeignete Apparaturen während des Extrudierens Wirkstoffe und weitere Zusätze hinzuzufügen. Probleme bereitet es hierbei jedoch auch heute noch, eine wirklich homogene Wirkstoffvermischung zu erzielen und ein Produkt mit wirklich glatter Oberfläche zu erhalten.

Versuche haben gezeigt, daß sich Zusammensetzungen auf der Basis von Sorbit, Xylit, Lactit oder anderen zuckeranalogen Substanzen, wie Maltit, Erythrit, Mannit oder anderen, die gegebenenefalls zusätzlich Kohlenhydrate aus der Gruppe Stärke, Cellulose enthalten können, in einfacher Weise zu Extrudaten formen lassen, die gut und lange weiterverarbeitbar sind. Auch Zusammensetzungen, die diese Polyole im Gemisch enthalten, sind in gleicher Weise und gut verarbeitbar.

Es wurde auch gefunden, daß polyolhaltige Massen, die einen hohen Xylitgehalt besitzen, sich besonders gut verarbeiten lassen. Überaus gute Verarbeitungseigenschaften weisen insbesondere solche Massen auf, deren Komponenten vor dem Extrudieren in dem in der Patentanmeldung DE 19617487.2 beschriebenen Co-Sprühverfahren vorbehandelt und miteinander zu einem feinteiligen Pulver verarbeitet worden sind. Es handelt sich bei diesem zur Extrusion eingesetzten Pulver nicht nur um eine Mischung von zwei oder mehreren verschiedenen Pulvern sondern um ein Pulver, worin bereits die einzelnen Partikel aufgrund der Co-Versprühung aus einem Gemisch der Einzelkomponenten bestehen, d. h. es werden Mischkristalle erhalten. Diese Pulver weisen im Vergleich zu üblicherweise verwendeten Pulvermischungen einen niedrigeren Schmelzpunkt auf, und hieraus erhaltene plastische Massen sind auch nach dem Extrudieren lange und gut verformbar. Diese Verformbarkeit kann durch die Zugabe von geeigneten, dem Fachmann bekannten, Kristallisationsverzögerern, die während des Co-Versprühens hinzugefügt werden können, noch verlängert werden. Vorteilhafterweise kann bei Zusammensetzungen auf der Basis erfindungsgemäßer co-gesprühter Polyole oder Polyolgemische auf die Zugabe sonst notwendiger Weichmacher oder Fließregulierungsmittel verzichtet werden.

Durch das vorgeschaltete Co-Sprüh-Trocknen der Einzelkomponenten werden Pulver erhalten, die, so wie sie im Co-Sprühverfahren entstehen und gesammelt werden, kontinuierlich extrudiert werden können. Zwischen den oder während der Verarbeitungsstufen des Co-Versprühens und des Extrudierens können den Pulvermischungen Wirkstoffe, Zusätze und übliche pharmazeutische Hilfsstoffe, wie Füllstoffe, Schmiermittel, Formentrennmittel, Fließregulierungsmittel, Weichmacher, Farbstoffe, Stabilisatoren, Säuerungsmittel, Geschmacks- und Aromastoffe hinzugefügt werden.

Als Füllstoffe können die dem Fachmann allgemein bekannten, wie Oxide des Magnesiums, Aluminiums, Siliziums und Titans aber auch andere hinzugefügt werden.

Bei Bedarf können in bestimmten Fällen geeignete Fließregulierungsmittel wie z. B. Mono-, Di- und Triglyceride der langkettigen Fettsäuren, Wachse, Camaubawachs, oder Lecithine hinzugefügt werden. Im allgemeinen werden in den erfindungsgemäßen Zusammensetzungen diese Zusätze jedoch nicht benötigt.

Neben niedermolekularen Polyalkylenoxiden wie Polyethylenglykol, Polypropylenglykol und Polyethylenpropylenglykol sind auch mehrwertige Alkohole wie Propylenglykol, Glycerin, und Pentaerythrit sowie Natriumdiethylsulfosuccinat, Mono-, Di- und Triacetat des Glycerins und Polyethylenglykolstearinsäureester als Weichmacher geeignet, die falls notwendig hinzugefügt werden können.

Als Schmiermittel können Stearate des Aluminiums oder Calciums sowie Talkum oder Silikone dienen.

Als Farbstoffe können natürliche Färbemittel ebenso eingesetzt werden wie alle als Lebensmittelzusatzstoff zugelassenen Farbstoffe und Pigmente.

Als Stabilisatoren kommen in Frage Antioxidantien, Radikalfänger, Stabilisatoren gegen mikrobiellen Befall und Lichtstabilisatoren.

Je nach Zusammensetzung können alle Zusätze in den dem Fachmann geläufigen Konzentrationen zugesetzt werden, und zwar in solchen Konzentrationen, daß der jeweilige gewünschte Effekt des Zusatzes erzielt wird.

Es ist möglich, alle Zusätze während des Extrudierens hinzuzufügen. Vorteilhaft ist es jedoch, für eine gleichmäßige Verteilung im Produkt, lösliche Zusätze während des Co-Sprühverfahrens der Zusammensetzung zuzugeben. Unlösliche Zusätze können mit dem durch das Co-Versprühen erhaltene Pulver und gegebenenfalls den übrigen Komponenten mechanisch vor dem Extrudieren vermischt werden.

Durch das vorgeschaltete Co-Versprühen werden Mischungen erhalten, die mit einem hohen Durchsatz zu verformbaren Massen extrudiert werden können. Diese Pulvermischungen erfordern dabei einen geringeren Energieeintrag aufgrund des niedrigeren Schmelzpunktes und der verbesserten Plastifizierbarkeit, offensichtlich hervorgerufen durch eine veränderte Struktur der eingesetzten Pulver.

Je nach dem eingesetztem Polyol oder Polyolgemisch ist während des Extrudierens also ein bestimmter Energieeintrag erforderlich. Abhängig ist dieser, wie angedeutet, also von der Art und Weise, wie die Polyolgemische erhalten worden sind, und zwar durch einfaches Vermischen oder Co-Versprühen. Der Energieeintrag kann mechanisch durch den Extrudiervorgang und den dabei einwirkenden Kräften erfolgen. Er kann aber auch thermisch durch zusätzliches Erwärmen erfolgen. Hierbei gibt es spezifische Unterschiede, wobei die geringste Energiemenge bei den cogesprühten Polyolzusammensetzungen erforderlich ist. Die erfindungsgemäßen Polyolmischungen können im Temperaturbereich von 30 bis 170 °C, insbesondere von 40 bis 110 °C extrudiert werden. Als besonders geeignet haben sich für durch Co-Versprühen erhaltene Mischungen Bedingungen erwiesen, unter denen der Energieeintrag zu einer Produkttemperatur von etwa 70 bis 110 °C führt.

Während sich co-gesprühte Polyolmischungen in einfacher Weise extrudieren lassen, ist die Extrusion von mechanisch gemischten Polyolkombinationen zu homogenen Produkten nicht möglich, da die verschiedenen Polyole unterschiedliche Schmelzpunkte aufweisen. Insbesondere nicht co-gesprühte Mischungen, die Mannit enthalten, erfordern einen hohen Energieeintrag, da sonst ein grobkörniges Extrusionsprodukt erhalten wird, in dem die Mannitkristalle als solche vorliegen. Für mannithaltige Mischungen empfiehlt sich daher ein vorgeschaltetes Co-Versprühen. Durch anschließende Extrusion, wobei der Energieeintrag zu einer Produkttemperatur von bis zu 110 °C am Austrittsort führt, werden nach dem Formen Produkte mit glatter Oberfläche und einem guten Lutschverhalten erhalten. Nach dem erfindungsgemäßen Verfahren verarbeitete mannithaltige Mischungen weisen besonders gute Produkteigenschaften auf, wenn darin mindestens zwei weitere Polyole in einer Menge von bis zu 10 Gew-% enthalten sind.

Durch Vergleichsversuche wurde generell gefunden, daß vorher co-gesprühte Polyolzusammensetzungen zu Extrusionsprodukten mit glatterer Oberfläche verarbeitet werden können als wenn einfach vermischte Zusammensetzungen zur Extrusion eingesetzt werden. Wird beispielsweise ein im Handel erhältliches sprühgetrocknetes Sorbit (Karion Instant®) als Hauptkomponente nach einfachem Vermischen mit den übrigen Komponenten der Zusammensetzung direkt extrudiert, wird nach dem Extrudieren, wobei das austretende Produkt eine Temperatur von ca. 100 °C aufweist, und dem anschließenden Formen ein Produkt mit einer rauheren Oberfläche erhalten als wenn eine entsprechende vorher co-gesprühte Zusammensetzung verwendet wird. Wird dagegen ein anderes, ebenfalls im Handel erhältliches kristallines Sorbit (Neosorb®) in gleicher Weise behandelt, werden Extrudate mit glatten Oberflächen erhalten, die zusätzlich ein gutes Lutschverhalten zeigen. Es ist hierbei jedoch ein sehr hoher Energieeintrag notwendig, und es können nicht beliebig dicke Stangdurchmesser gewählt werden.

Je nach der Zusammensetzung der Mischungen und ihrer Vorgeschichte ist daher die Wahl eines bestimmten Extrusionsverfahrens angezeigt. Es kann mit Doppelschneckenextrudern oder Plastifizierschnecken gearbeitet werden, bei denen der Energieeintrag über die Schnecke und gegebenenfalls zusätzlich durch Erwärmung erfolgen kann. Es kann aber auch unter Erwärmung mit einer Kompaktieroder Förderschnecke extrudiert werden. Als variable Parameter während des Extrudierens können u. a. die Produkteintragsmenge, die Fördergeschwindigkeit der Schnecke, die Größe der Austrittsdüse und die Temperatur geändert werden.

Die nach vorherigem Co-Versprühen durch das erfindungsgemäße Verfahren erhaltenen Massen lassen sich aufgrund ihrer guten Verarbeitungseigenschaften mit gutem Ergebnis durch größere Hohldurchmesser pressen als üblicherweise für entsprechende Produkte verwendet werden. Hierdurch wird ein höherer Produktdurchsatz erzielt.

Mit besonders guten Ergebnissen lassen sich nach dem erfindungsgemäßen Verfahren co-gesprühte Polyole verarbeiten aus der Gruppe Xylit, Sorbit, Lactit, Maltit, Erythrit und Mannit, deren Gemische oder Gemische mit anderen Polyolen, wobei ein oder mehrere dieser Polyole im Gemisch durch Extrusion zu einer plastischen, geformten oder ungeformten Masse verarbeitet werden. Als besonders geeignet haben sich Zusammensetzungen erwiesen, in denen die Polyole Sorbit und Xylit in einem Mengenverhältnis von 50 : 50 bis 99 :1, insbesondere von 65 : 35 bis 98:2, enthalten sind. Zusammensetzungen, in denen die drei Polyole Sorbit, Xylit und Mannit im Gemisch einhalten sind weisen besonders gute Eigenschaften auf, wenn diese in Mengenverhältnissen von 90 : 1 : 9 bis 70 : 29 : 1, insbesondere von 82 : 9 : 9, enthalten sind. Polyolgemischen dieser Zusammensetzung können vor dem Extrudieren verschiedenste Zusätze hinzugefügt sein. Solche Zusätze können beispielsweise ein oder mehrere Wirkstoffe, ein oder mehrere als Lebensmittelzusatz zugelassene Farbstoffe, aber auch ein oder mehrere natürliche und/oder ein oder mehrere synthetische Süßstoffe sein. Diese Zusätze können allein oder gemeinsam zugesetzt sein. Weiterhin können in der Pharma- oder Lebensmittelindustrie übliche Verarbeitungshilfen und Zusätze hinzugefügt sein. Diese Zusätze können unter Einsatz moderner Dosierwaagen, wie in EP-B1-0 337 256 beschrieben, konstant in gleichbleibender Dosierung hinzugefügt werden, so daß eine immer gleichbleibende Zusammensetzung extrudiert wird.

Die nach dem Extrudieren aus den erfindungsgemäßen Polyolzusammensetzungen erhaltenen plastischen geformten oder ungeformten Massen lassen sich durch üblicherweise in der Lebensmittel- oder Pharmaindustrie gebräuchlichen nachgeschaltete formgebende Anlagen, wie z. B. Prägewalzen oder Rollautomaten weiterverarbeiten.

Aus den erfindungsgemäßen Massen hergestellte Produkte, wie Tabletten, Komprimate, Lutschtabletten oder -bonbons besitzen im Vergleich zu in bekannter Weise hergestellten Produkten eine wesentlich glattere Oberfläche auf, die auch während des Verzehrs und insbesondere beim Lutschen erhalten bleibt. Das Lösen im Mund erfolgt viel gleichmäßiger, wobei die ursprünglich sehr glatte Oberfläche auch erhalten bleibt. Die Bildung von scharfen Kanten ist durch diese verbesserten Eigenschaften stark reduziert. Besonders ausgeprägt sind diese vorteilhaften Eigenschaften bei Produkten deren Einzelkomponenten vor dem Extrudieren durch Co-Versprühen miteinander vermischt worden sind. Auch sind co-gesprühte Polyolzusammensetzungen aufgrund ihres niedrigeren Schmelzpunktes für das erfindungsgemäße Extrusionsverfahren besonders gut geeignet, da eingearbeitete Wirkstoffe, Aromen usw. einer geringeren Temperaturbelastung ausgesetzt werden und der Extrusionsstrang länger als üblich noch verformbar ist; und zwar bleibt das erhaltene Produkt nach dem Extrudieren noch für etwa 1 bis zwei Minuten plastisch, weich und verformbar. Weiterhin ist man im Vergleich zu üblicherweise erhaltenen Extrudaten in der Wahl des Strangdurchmessers am variabelsten aufgrund des ausgeprägten und guten plastischen Verhaltens der erfindungsgemäßen Extrudate. Auch zeigen diese Zusammensetzungen nach dem Extrudieren und Formen das besseres Lutschverhalten als bisher bekannt.

Die erfindungsgemäße Verfahren weist gegenüber den herkömmlichen eine Reihe von Vorteilen auf. Hierzu zählt u. a. die Möglichkeit kontinuierlich pulverförmige Zusammensetzungen mit fortwährend gleichen Konzentrationen der Einzelkomponenten herstellen zu können, welche direkt unter schonenderen Bedingungen zu plastischen geformten oder ungeformten Massen extrudiert werden und in einem nachgeschalteten Verfahren zu Tabletten, Komprimaten, Lutschtabletten oder -bonbons geformt werden können. Ein besonderer Vorteil der erfindungsgemäßen Zusammensetzungen ist, daß die hergestellten Produkte weitaus glattere Oberflächen aufweisen, die auch während des Lutschens erhalten bleiben. Weiterhin wird durch das vorgeschaltete Co-Versprühen mit den erfindungsgemäßen Polyolen und gegebenenfalls mit Mannit eine vorteilhafte Geschmacksverbesserung erzielt. Insbesondere ein kreidiger Geschmack, wie er bei bekannten Antacida auftritt, wird durch die erfindungsgemäßen Zusammensetzungen überdeckt. Auch wird hierdurch die Bioverfügbarkeit und Resorption von eingearbeiteten Wirkstoffen gesteigert, da die hergestellten Tabletten aufgrund der verwendeten Trägersubstanzen leicht löslich sind und aufgrund ihres angenehmen Geschmacks ohne weiteres gelutscht werden können. Dieses ist von Bedeutung, wenn eine schnelle Wirksamkeit erwünscht ist, wie dieses beispielsweise bei enthaltenen Analgetika der Fall sein kann.

Falls gewünscht kann die erfindungsgemäß hergestellte Tablette auch mit einem üblichen Überzug zur Verbesserung des Aussehens oder zwecks zusätzlicher Verzögerung der Wirkstoffabgabe versehen werden. Es kann günstig sein für Tabletten mit verzögerter Wirkstoffabgabe, wenn man die Tablette nach einer der bekannten Techniken in geschlossenzellig poröser Form herstellt, damit sie im Magen aufschwimmt und dadurch länger verweilt.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. So kann die Wirkstoffkonzentration im Bereich von 0,1 bis 95 , vorzugsweise von 5 bis 80, liegen. Auch Wirkstoffkombinationen können eingesetzt werden. Die erfindungsgemäßen Zusammensetzungen sind vorzugsweise zur Einarbeitung von solchen Wirkstoffen geeigent, deren sofortige biologische Verfügbarkeit erwünscht ist und die gemeinsam mit den übrigen Bestandteilen ein vorteilhaftes Geschmackprofil ergeben. Solche Wirkstoffe können Antacida, Analgetika, Sedativa, Relaxantien oder andere pharmazeutische Wirkstoffe sein. Wirkstoffe im Sinne der Erfindung sind auch ernährungsphysiologische Substanzen, wie Vitamine, Mineralstoffe und Spurenelemente.

Die im folgenden gegebenen Beispiele sollen der Veranschaulichung der vorliegenden Erfindung dienen.

### Beispiele

### 1. Versuchsaufbau und Durchführung

- Anlage:: Continua 37, Firma Wemer & Pfleiderer, Stuttgart
Leistung: max. 400 Upm und 7,6 KW
Standardgerät mit 6 Gehäusen
Einlaufgehäuse mit Wasser gekühlt
Gehäuse 2-4 und 5-6 geschlossen, getrennt temperierbar
Feststoffeintrag über Doppelschneckendosierung Arbo KDS-VS 26
Cerealien- als auch Förderschnecken
Austrag über verschiedene Düseneinsätze

### Versuchdurchführung:

Nach Temperierung der Heizzonen wurde trocken angefahren. Danach wurden Temperatur und Leistung variiert.

### 2. Zusammensetzung der Ausgangsmaterialien

a) Sorbit > 91%
   Xylit > 4%
   Mannit > 3%
   Herstellung durch Copsprühung
b) gleich Zusammensetzung, jedoch mechanische Mischung
c) Sorbit, sprühgetrocknet
d) Sorbit, kristallisiert
e) Zusammensetzung a 80%
   Kaliumchlorid 20%
f) Zusammensetzung d 80%
   Kaliumchlorid 20%
g) Zusammensetzung a 50%
   Ascorbinsäure 50%
h) Zusammensetzung d 50%
   Ascorbinsäure 50%
i) Zusammensetzung a 80%
   Acetylsalicylsäure 20%
k) Zusammensetzung 60%
   Magnesiumcitrat 40%

**Tabelle 1:**

| Trägerstoffe | | | | |
|---|---|---|---|---|
| Zusammensetzung | a | b | c | d |
| Extrusionsdüse | 1x4,5 | 1x4,5 | 1x4,5 | 1x4,5 |
| Extrudertemperat ur (°C) Zone 2-4 | 80 | 90 | 110 | 115 |
| Extrudertemperat ur (°C) Zone 5-6 | 80 | 90 | 110 | 115 |
| Produkttemperat ur an der Austrittsdüse (°C) | 92 | 90 | 100 | 103 |
| Aushärtezeit des Strangs (sec) | 40 | 25 | 25 | 20 |
| Aussehen des Stranges | weiß, sehr glatte Oberfläche | weiß, körnige Oberfläche, teilweise brüchig | weiß, rauhe Oberfläche | weiß, rauhe Oberfläche |
| Sensorik | angenehme Süße | schwach süß, rauhes Lutschverh alten | geringere Süße als bei a), leicht rauhes Lutschverha Iten | geringere Süße als bei a), leicht rauhes Lutschverh alten |
| Veränderung der Kirstallinität (DSC) | keine | nicht bestimmt | keine | keine |
| Bemerkungen | Material ist für Austrittsdüse oval, 12x9 geeignet | Material ist für Extrusion nicht geeignet | bei Austrittsdüse oval, 12x9 bröseliger, offen poriger Strang | bei Austrittsdüse oval, 12x9 bröseliger, offen poriger Strang |

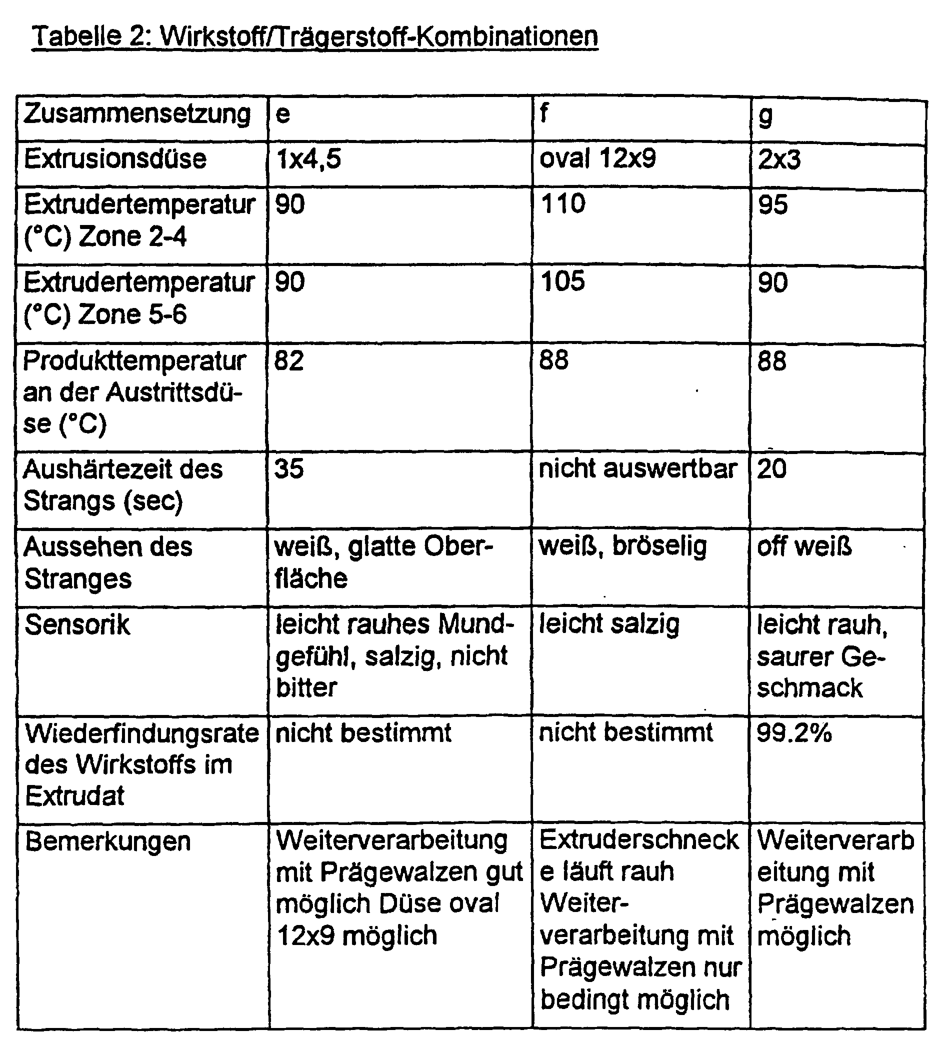

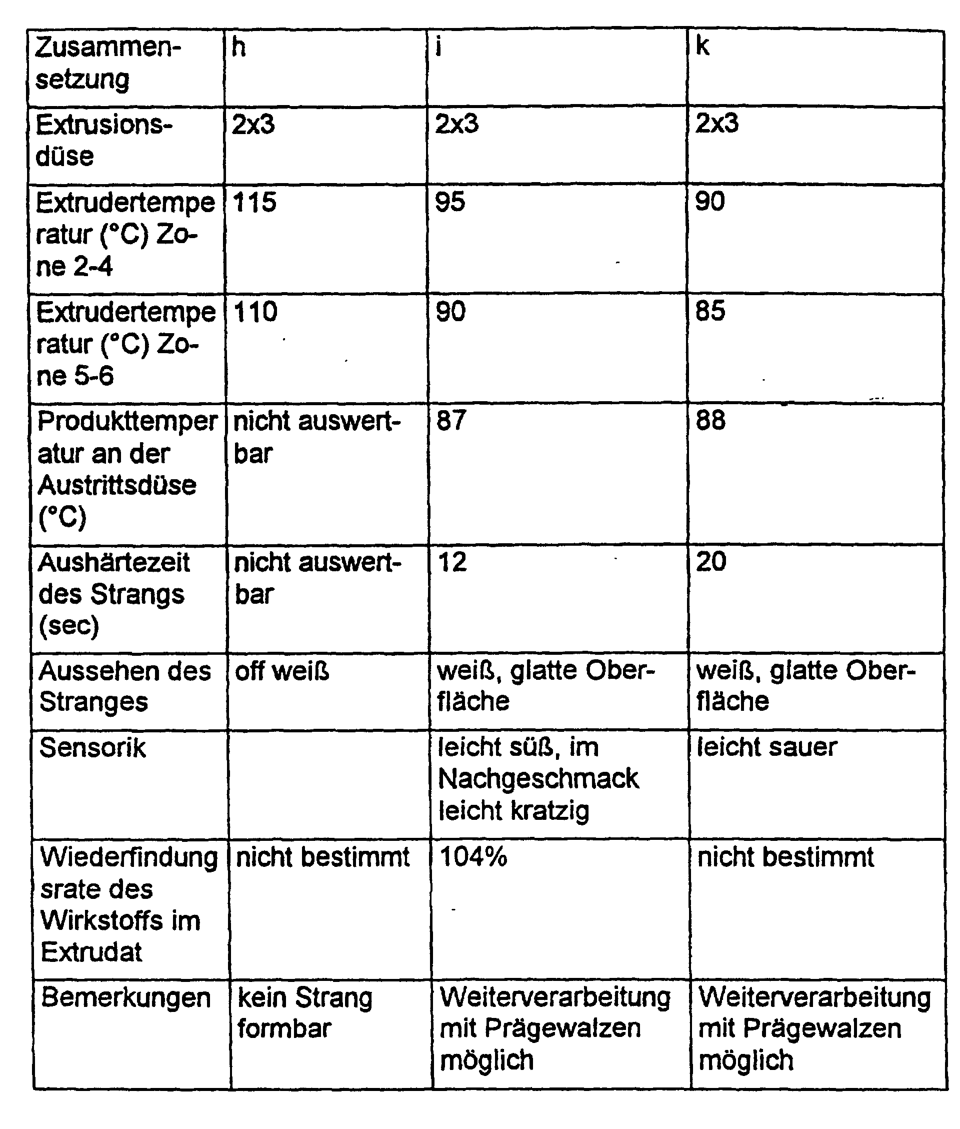

## Patentansprüche

1. Zusammensetzung zur Herstellung von Tabletten, Komprimaten oder Lutschbonbons, enthaltend mindestens zwei Polyole, sowie gegebenenfalls ein oder mehrere Kohlenhydrate/e aus der Gruppe Stärke und Cellulose, gegebenenfalls einen oder mehrere Wirkstoffe, einen oder mehrere Farbstoffe, einen oder mehrere natürliche Süßstoffe, einen oder mehrere synthetische Süßstoffe, Säuerungsmittel, Geschmacksstoffe, Aromastoffe, sowie übliche Hilfstoffe,
herstellbar durch
a) Co-Versprühen von mindestens zwei Polyolen, gegebenenfalls mit löslichen Zusätzen, und
b) Extrusion in einem Temperaturbereich von 40 bis 110 °C in der gewünschten Zusammensetzung zu plastischen formbaren Massen, wobei
c) die Polyolzusammensetzungen entweder vor oder während des Extrudierens mit den unlöslichen Komponenten vermischt werden.

2. Zusammensetzung nach Anspruch 1, enthaltend mindestens zwei Polyole aus der Gruppe Xylit, Sorbit, Lactit, Maltit, Erythrit oder Mannit.

3. Zusammensetzung nach einem oder mehreren der Ansprüche 1 bis 2, enthaltend die beiden Polyole Sorbit und Xylit in einem Mengenverhältnis von 50 : 50 bis 99 : 1, insbesondere von 65 : 35 bis 98 : 2.

4. Zusammensetzung nach einem oder mehreren der Ansprüche 1 bis 3, enthaltend die drei Polyole, Sorbit, Xylit, Mannit, in einem Mengenverhältnis von 90 : 1 :9 bis 70 : 29 : 1, insbesondere von 82 : 9 : 9.

5. Tabletten, Komprimate oder Lutschtabletten oder -bonbons, enthaltend eine Zusammensetzung gemäß einem oder mehreren der Ansprüche 1 bis 4.

6. Wirkstoffhaltige Tabletten, Komprimate, Lutschtabletten oder - bonbons gemäß Anspruch 5.

7. Verfahren zur Herstellung einer Zusammensetzung gemäß einem oder mehreren der Ansprüche 1 bis 4, **dadurch gekennzeichnet, daß** mindestens zwei Polyole,welche zuvor, gegebenenfalls in Gegenwart von löslichen Zusätzen,cogesprüht worden sind, und gegebenenfalls ein oder mehrere Kohlenhydrate, gegebenenfalls ein oder mehrere Wirkstoffe, ein oder mehrere Farbstoffe, ein oder mehrere natürliche Süßstoffe, ein oder mehrere synthetische Süßstoffe, Säuerungsmittel, Geschmacksstoffe, Aromastoffe, Hilfstoffe
in einem Temperaturbereich von 30 bis 170 °C, insbesondere in einem Bereich von 40 bis 110 °C extrudiert und gegebenenfalls die durch Extrudieren hergestellte plastische Masse, welche in Form eines Stranges vorliegen kann, in nachgeschalteten formgebenden Anlagen geformt werden und weiterverarbeitet wird.

8. Verfahren gemäß Anspruch 7, **dadurch gekennzeichnet, dass** während des Co-Versprühens Wirkstoffe hinzugefügt werden.

9. Verfahren gemäß der Ansprüche 7 bis 8, **dadurch gekennzeichnet, daß** eine durch Co-Versprühen erhaltene Zusammensetzung bei einer Temperatur unterhalb von 110 °C extrudiert wird.

10. Verfahren gemäß Anspruch 9, **dadurch gekennzeichnet, daß** die Formgebung auf Prägewalzen oder in Rollautomaten erfolgt.

11. Verfahren nach einem oder mehreren der Ansprüche 7 bis 10, **dadurch gekennzeichnet, daß** Zusammensetzungen verwendet werden, denen durch mechanisches Mischen vor dem Extrudieren oder kontinuierlich ein oder mehrere Wirkstoffe, gegebenenfalls ein oder mehrere Farbstoffe und/ oder ein oder mehrere natürliche und/ oder ein oder mehrere synthetische Süßstoffe, Säuerungsmittel, Geschmacksstoffe; Aromastoffe und gegebenenfalls Kristallisationsverzögerer zugesetzt werden.

## Claims

1. Composition for the production of tablets, compacts or boiled sweets, comprising at least two polyols, and optionally one or more carbohydrates from the group consisting of starch and cellulose, optionally one or more active compounds, one or more colourants, one or more natural sweeteners, one or more synthetic sweeteners, acidifying agents, flavourings, aromatizers and customary auxiliaries,
preparable by
a) co-spraying at least two polyols, optionally with soluble additives, and
b) extruding in a temperature range from 40 to 110°C in the desired composition to form plastic shapable materials, where
c) the polyol compositions are mixed either before or during extrusion with the insoluble components.

2. Composition according to Claim 1, comprising at least two polyols from the group consisting of xylitol, sorbitol, lactitol, maltitol, erythritol and mannitol.

3. Composition according to one or both of Claims 1 and 2, comprising the two polyols sorbitol and xylitol in a quantitative ratio of 50:50 to 99:1, in particular of 65:35 to 98:2.

4. Composition according to one or more of Claims 1 to 3, comprising the three polyols sorbitol, xylitol and mannitol in a quantitative ratio of 90:1:9 to 70:29:1, in particular of 82:9:9.

5. Tablets, compacts or lozenges or boiled sweets, comprising a composition according to one or more of Claims 1 to 4.

6. Tablets, compacts, lozenges or boiled sweets according to Claim 5, comprising an active compound.

7. Process for the preparation of a composition according to one or more of Claims 1 to 4, **characterized in that** at least two polyols which have been co-sprayed beforehand, optionally in the presence of soluble additives, and optionally one or more carbohydrates, optionally one or more active compounds, one or more colourants, one or more natural sweeteners, one or more synthetic sweeteners, acidifying agents, flavourings, aromatizers, auxiliaries are extruded in a temperature range from 30 to 170°C, in particular in a range from 40 to 110°C, and optionally the plastic material produced by extrusion, which may be present in the form of a rod, is shaped in subsequent shaping units and is processed further.

8. Process according to Claim 7, **characterized in that** active compounds are added during the co-spraying.

9. Process according to Claims 7 and 8, **characterized in that** a composition obtained by co-spraying is extruded at a temperature below 110°C.

10. Process according to Claim 9, **characterized in that** the shaping is carried out on engraved rollers or in automatic rolling machines.

11. Process according to one or more of Claims 7 to 10, **characterized in that** compositions are used to which one or more active compounds, if appropriate one or more colourants and/or one or more natural and/or one or more synthetic sweeteners, acidifying agents, flavourings, aromatizers and, if appropriate, crystallization retardants are added by mechanical mixing before extrusion or continuously.

## Revendications

1. Composition pour la fabrication de comprimés, de préparations compressées, ou de bonbons à sucer, contenant au moins deux polyols, ainsi qu'éventuellement un ou plusieurs hydrates de carbone du groupe amidon et cellulose, éventuellement une ou plusieurs substances actives, un ou plusieurs colorants, un ou plusieurs édulcorants naturels, un ou plusieurs édulcorants synthétiques, des acidifiants, des agents donnant du goût, des aromatisants, ainsi que des adjuvants habituels, préparés par
a) co-pulvérisation d'au moins deux polyols, éventuellement avec des additifs liquides, et
b) extrusion dans une plage de températures comprise entre 40 et 110°C dans la composition désirée pour obtenir une masse plastique malléable,
c) les compositions de polyols étant mélangées avec les composants insolubles avant ou pendant l'extrusion.

2. Composition selon la revendication 1, contenant au moins deux polyols du groupe xylite, sorbite, lactite, maltite, érythrite ou mannite.

3. Composition selon la revendication 1 ou 2, contenant les deux polyols sorbite et xylite dans un rapport quantitatif compris entre 50:50 et 99: 1, en particulier entre 65:35 et 98:2.

4. Composition selon une ou plusieurs des revendications 1 à 3, contenant les trois polyols sorbite, xylite et mannite dans un rapport quantitatif compris entre 90:1:9 et 70:29:1, en particulier dans un rapport 82:9:9.

5. Comprimés, préparations compressées ou pastilles ou bonbons à sucer, contenant une composition selon une ou plusieurs des revendications 1 à 4.

6. Comprimés, préparations compressées, pastilles ou bonbons à sucer contenant de la substance active selon la revendication 5.

7. Procédé pour la préparation d'une composition selon une ou plusieurs des revendications 1 à 4, **caractérisé en ce que** l'on extrude au moins deux polyols, préalablement co-pulvérisés, éventuellement en présence d'additifs solubles, et éventuellement un ou plusieurs hydrates de carbone, éventuellement une ou plusieurs substances actives, un ou plusieurs colorants, un ou plusieurs édulcorants naturels, un ou plusieurs édulcorants synthétiques, des acidifiants, des agents donnant du goût, des aromatisants, des adjuvants dans une plage de températures comprise entre 30 et 170°C, en particulier dans une plage comprise entre 40 et 110°C et éventuellement **en ce que** l'on façonne la masse plastique obtenue par extrusion et pouvant se présenter sous la forme d'un boudin dans des installations de formage placées à la suite, et la transforme ultérieurement.

8. Procédé selon la revendication 7, **caractérisé en ce que** l'on ajoute des substances actives pendant la copulvérisation.

9. Procédé selon la revendication 7 ou 8, **caractérisé en ce que** l'on extrude une composition obtenue par co-pulvérisation à une température inférieure à 110°C.

10. Procédé selon la revendication 9, **caractérisé en ce que** le façonnage est réalisé sur des rouleaux graineurs ou dans des machines enrouleuses automatiques.

11. Procédé selon une ou plusieurs des revendications 7 à 10, **caractérisé en ce que** l'on utilise des compositions dans lesquelles l'on additionne par mélange mécanique avant l'extrusion ou, de façon continue, une ou plusieurs substances actives, éventuellement un ou plusieurs colorants et/ou un ou plusieurs édulcorants naturels et/ou un ou plusieurs édulcorants synthétiques, des acidifiants, des agents donnant du goût, des aromatisants et éventuellement des retardateurs de cristallisation.
